# EUROPEAN PATENT SPECIFICATION

(11) **EP 2 319 392 B1**
(45) Date of publication and mention of the grant of the patent: **27.08.2014**
(21) Application number: 09800082.1
(22) Date of filing: 13.03.2009
(51) Int. Cl.: A61B 3/107, A61B 5/117, A61B 3/10, G06K 9/00

(54) **BIOMETRIC RECOGNITION THROUGH EXAMINATION OF THE SURFACE MAP OF THE POSTERIOR SURFACE OF THE CORNEA**
BIOMETRISCHE ERKENNUNG MITTELS UNTERSUCHUNG DER OBERFLÄCHENKARTE DER HINTEREN FLÄCHE DER HORNHAUT
RECONNAISSANCE BIOMÉTRIQUE FONDÉE SUR UNE ÉTUDE DE LA CARTE TOPOGRAPHIQUE DE LA SURFACE POSTÉRIEURE DE LA CORNÉE

(30) Priority: 24.07.2008 ES 200802215
(43) Date of publication of application: 11.05.2011
(73) Proprietor: Universidad Complutense de Madrid, 28040 Madrid (ES)
(72) Inventor: SÁNCHEZ RAMOS, Celia, E-28037 Madrid (ES)
(74) Representative: Temino Ceniceros, Ignacio
(86) International application number: PCT/ES2009/000145
(87) International publication number: WO 2010/010203

(56) References cited:
- WO-A1-03/065290
- WO-A1-2006/034527
- WO-A1-2007/104166
- US-A1- 2007 236 664
- DUBBELMAN M. ET AL.: 'The shape of the anterior and posterior surface of aging human cornea' VISION RESARCH vol. 46, no. 6-7, 01 March 2006, OXFORD, GB, pages 993 - 1001, XP025010062
- DATABASE BIOSIS [Online] 2004 DUBBELMAN M. ET AL.: 'Radius, astigmatism and asphericity of the posterior corneal surface', XP008142726 Database accession no. 200510250677
- KOPACZ, D.: 'Pentacam-the new way for anterior eye segment imaging and mapping' KLINIKA OCZNA vol. 107, no. 10-12, 2005, XP008142512

## Description

### FIELD OF THE INVENTION

The invention is part of the biometric sector, applied to security and recognition of persons. The invention refers specifically to a method and device for biometric recognition of the second optical dioptric.

The object of the invention is a method and the corresponding device thereof for recognition of persons incorporating, as biometric constant and determinant for each individual, the irregularities of the map of the posterior surface of the cornea, being different in each individual.

### STATE OF THE ART

The different types of biometric person recognition systems are based on the physical characteristics of the user to be identified. Although the identification of users via biometric methods is possible using any unique and measurable characteristic of the individual, it has traditionally been based on six large groups: eye-iris, eye-retina, fingerprints, geometry of the hand, writing-signature and voice.

Biometric authentication models based on ocular patterns are divided into two different technologies: they either analyze retina patterns or they analyze the morphological characteristics of the iris.

The iris is the most visible part of the human eye. It is a complex pattern that contains many different features such as wrinkles, arched ligaments, crests or rings. The image of the iris can be taken from a reasonable distance (1m) with a great level of accuracy. To accomplish this requires a camera with a minimum resolution of 70 pixels. Although the level of accuracy of the image is high and the processing time is long, it is difficult to obtain if the person does not stay close to the camera or does not want to be identified. Also, medical procedures can somewhat alter the colour (although the texture is maintained stable for decades), the iris is humid, which is why it can be reflected, it can be covered and can be manipulated using contact lenses and suffers modifications in size due to the dilation of the pupil.

Retina scanning is a biometric technique that uses the patterns present in the retina to identify a person. Authentication via the retina can be carried out through the initial recording of the vascular structure of the retina (shape of the blood vessels of the human retina), which has characteristic elements of each individual, which are different from the rest of the population.

In these systems, the user to be identified must look through an ocular device, adjust the distance and the movement of the head, look at a determined fixed point and, finally, press a button to tell the device that he is ready for the analysis. In order to obtain a valid record, you must wait five minutes for the retina mydriasis or dilation to occur, which is required in systems that enter through the pupil, or use mydriatic drugs. Subsequently, the retina is scanned using spiral shaped low intensity infrared radiation; retina nodes and branches are detected in an image for comparing them with those stored in a database; if the sample coincides with that stored for the user the individual claims to be, the authentication is then validated.

These methods are typically considered more effective for a population of millions of potential users since the probability of coincidence between individuals is practically null. An additional characteristic of great importance in the authentication process is the fact that once the individual is dead, the ocular tissue degenerates rapidly, which makes it difficult for a false identification of intruders that can steel this organ from a corpse in order to falsify an authentication. Other patents related with this technique are known. Thus, Patent US 4,109,237 describes a device for identifying individuals through the vascular pattern of the retina. Document US 4,487,322 describes a device to measure the level of oxygen in the blood at the bottom of the eyeball; US2004233038 describes a system and a method for retina recognition at ports of embarkation. Documents KR20030034258, WO02075639, WO2007023946, WO2006073781, US2001022850 and GB1599015 also include different systems for capturing images from the retina or the iris.

However, the recognition through the iris and the retina include similar limitations and new systems and methods are still required in order to overcome these current limitations.

Recently, biometric authentication models based on ocular patterns, which are centred on the cornea as a pattern, have been developed. Thus, document US2007/0291997 "Corneal Biometry apparatus and method", describes an apparatus and method for validating the identity of a person, using imaging techniques of the cornea, which assesses the cornea as a set, therefore measuring parameters such as the corneal radius and the dioptric power. These variables change with time, with disease, surgery, the presence of a lens, etc. and, above all, they can be externally manipulated, for example, using a contact lens. Document US2006/0210122 describes a method and system based on the topography of the surface of the eyeball (for the external face of the cornea as well as the sclera) to identify a user, which is based on detecting changes to the aforementioned surface, measuring the reflection it produces as compared to the reflection that an ideal sphere would produce. This parameter can also be manipulated for example, using contact lenses.

Different devices exist for capturing the image of the cornea. Some of these are based on the Scheimpflug's rotating camera, which enables to capture a multi-sectional image of the back of the eye and scans the centre of the cornea with the same precision as the peripheral areas, independently of the tear film. Some well known devices are the PENTCAM^{®} (Oculus) and the ORBSCAN II^{®} (Bausch & Lomb Inc.), which enable to determine, in the first case, or calculate, using the second instrument, maps of the corneal surface elevation.

These elevation maps or topographies have been widely used to measure the density and thickness of the cornea as well as for determining the curvature radius and asphericity of the second ocular dioptric (for example, "Radius and asphericity of the posterior corneal surface determined by corrected Scheimpflug photography". Dubbelman, M. et al, Acta Ophtalmolo. Scand. 2002:80: 379-383*,* "Surface and Orbscan II slip -scanning elevation topography in circumscribed posterior Keratoconus" Charles et al. J. of Cataract and Refractive Surgery, 2005: 31(3): 636-639). These investigations are directed at studying the cornea to determine the change in the posterior surface after having undergone refractive surgery of the cornea or to determine if a patient is developing keratoconus or any other ectasia and study its evolution.

Further, the document "The shape of the anterior and posterior surface of the aging human cornea", Dubbelman, M. et al, Vision Research, vol. 46, no. 6-7, 1 March 2006, p. 993-1001) discloses a method and the associated device that encompasses capturing an image of the posterior surface of the cornea by corrected Scheimpflug photography, in order to study the effect of aging on the shape of the cornea in a healthy population.

For the purpose of overcoming the aforementioned inconveniences, this invention provides a biometric recognition method and a system based on the analysis of the irregularities of the posterior surface of the cornea with respect to the standardized surface, which has, as a main advantage, the impossibility of manipulating said map (since it is inside the eye), neither by an external individual, nor by the person to be recognized. Also, the method is non-invasive, fast, inexpensive, easy to use and based on a constant, which in addition to not being able to manipulate, is easy to record and is relatively stable in time, even after suffering disease or undergoing surgery.

The invention is based on taking an image, exclusively of the second ocular dioptric, for the purpose of determining the irregularities of the surface map thereof with respect to a standardized surface (sphere, ellipsoid, torus, etc.) and quantifying them, yielding a set of attributes characteristic of each person utilised as a system of authentication, that is to say, utilising the variations and calculations carried out therewith as biometric minutiae. Taking an image is of proven safety insofar as it may be taken without any contraindications whatsoever and as many times as necessary.

### DESCRIPTION OF THE INVENTION

The objective of the invention is a method and device for the recognition of persons, which incorporates as a biometric constant, the irregularities of the second ocular dioptric surface map.

The majority of ocular model studies introduce a simplification, considering that the posterior surface of the cornea is completely spherical. However, this invention includes the study exclusively of the second surface, thus determining the map of the specific surface.

The corresponding curvature has been measured through Purkinje images. The asphericity of the posterior surface can be determined by combining a videokeratoscope and a calliper, which leads to a measurement that is not very precise and is time consuming. However, Scheimpflug's camera allows recording the anterior and posterior surfaces of the cornea as well as its thickness in a single pass by means of real photography of this medium since it is transparent.

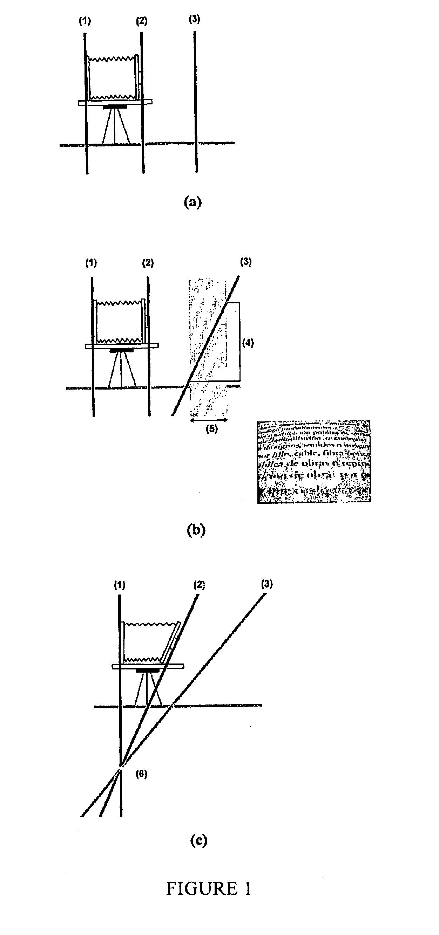

The optical fundamental of the recording is explained in the corresponding figures. Normally, the image plane, the lens plane and the object plane of a camera are parallel to each other (Figure 1a). If the object plane is not parallel to the image plane as occurs in eye photography, using a normal camera, only a small centre region is focused, which corresponds to a portion located within the depth of field (Figure 1b). In order to solve this problem, rotating cameras based on Scheimpflug's principle are used that allow capturing the entire object since it enables to achieve a wide depth of focus (Figure 1c).

The Scheimpflug principle is a geometric rule that describes the orientation of the plane of focus of an optic system when the lens plane is not parallel to the image plane.

This concept was presented by Carpentier (1901) in a British patent, in which he describes an extension to correct the vertical convergence. He researched the correlation between the inclination of the negative plane and the lens's optic axis, and he discovered that if the planes were extended, they should intersect a plane perpendicular to the axis and which passes by the optic centre of the lens. (Scheimpflug principle). According to this condition, an object that is not parallel to the image plane can be completely focused.

Based on this patent, Scheimpflug, in a prior patent (1904) studied the object that was being considered with the largest depth of field, describing not only the use of simple lenses, but also optical systems that are based on multifocals, mirrors and combination of lenses and mirrors to achieve corrections of distortions.

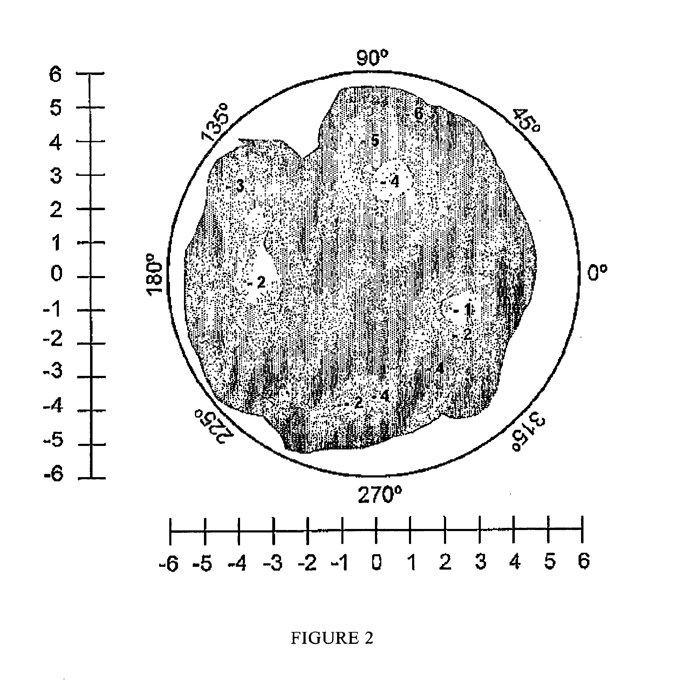

In the present invention, the recognition of the person is carried out through the capturing of an image of the posterior surface of the cornea in real time. The system contains a rotating Scheimpflug camera that records a precise three-dimensional image of the cornea. From this image, the maps of the first and second dioptric of the ocular optic system through a true elevation map are generated (Figure 2).

The photography obtained with the Scheimpflug camera is mathematically corrected considering the optic effect of the first dioptric in the image of the second photographed dioptric before proceeding with the analysis of the surface map of the second dioptric (Dubbelman & Van der Heide, 2001, Vision Research, 41*,* Dubbelman & Van der Heide, 2005, Vision Research, 45 and Dubbelman, Sicam & Van der Heide, 2006, Vision Research, 46 993-1001*).*

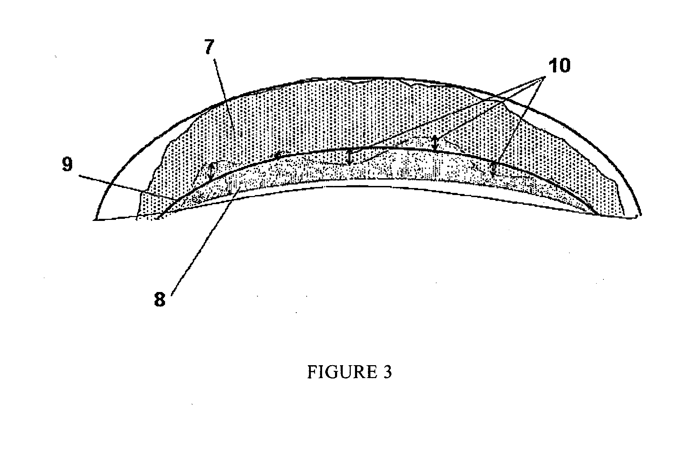

Finally, comparing the surface maps of the second ocular dioptric with a template surface (Figure 3), the characteristic and unique parameters of each individual are determined. To accomplish this, through a data analysis and processing system, these variations are quantified and those that are only characteristics of a specific individual and which differentiate them from the rest, are determined. This way, the irregularities will be equivalent to a "corneal fingerprint" and can be used as biometric minutiae.

Because a single Scheimpflug photography provides great information regarding the anterior eye segment, the biometric constant, object of this invention (the irregularities of the map of the second ocular dioptric surface), can be combined with other ocular parameters like the corneal radiuses or the dioptric power. Similarly, it can be combined with any other biometric constant that is based on the iris or retina. This way, if the system requires it, with a single photograph that does not require contact with the eye nor pupil dilation, which is carried out in a few seconds and does not use hazardous radiations, several biometric constants can be determined that complement the irregularities of the second dioptric, thus increasing the precision of the method.

### DESCRIPTION OF THE FIGURES

Figure 1 shows different arrangements of the image (1), lens (2) and object (3) planes. In case (a), the three planes are parallel: in (b), the object plane is not parallel to the image and lens planes, with a focused area (4) and a depth of field (5); in case (c), the planes have a common intersection point (6).
Figure 2 shows a colour map that represents the surface map of the second ocular dioptric.
Figure 3 shows a schematic of the measurement of the surface irregularities of the second ocular dioptric. When recording a portion of the outer face of the cornea (7) and the second ocular dioptric (8), the surface of the second ocular dioptric is compared to a standard surface (9), which determines the irregularities on the surface of the second ocular dioptric for each individual (10).

### EMBODIMENT METHOD FOR THE INVENTION

The invention is illustrated using the following example, which is not limiting in scope.

### Example 1.

Application of a user authentication recognition method used as a security system for access to an instrument or location.

First of all, an image is taken of the user's cornea using a Scheimpflug conventional camera coupled to the information processing device. The system transforms the biometric minutiae that had been previously selected for each individual in a code that has been encrypted using special software.

The encrypted code is sent via satellite, cable or other means to the instrument or location that requires the password to perform the authentication or identification of the person. It is then compared with the pre-established data in order to immediately proceed with the access authorization or the start of the use of the instruments that require user or individual recognition.

## Claims

1. Method for biometric recognition that encompasses (a) capturing an image of the posterior surface of the cornea and (b) quantifying the irregularities of a map of said posterior surface of the cornea with respect to a standardized surface for obtaining characteristic and differentiating parameters of the posterior surface of the cornea in each individual.

2. Biometric recognition methods in accordance with claim 1, combined with any other biometric recognition method through the use of ocular parameters extracted from a photographic record obtained to quantify the irregularities of the surface map of the second ocular dioptric.

3. Biometric recognition method in accordance with claim 2, where other ocular parameters are as relative to the cornea as corneal radiuses, dioptric power or the refractive power of the cornea, as well as to the retina or iris.

4. Biometric recognition device that comprises a Scheimpflug rotating camera and a system for quantifying irregularities of the posterior surface of the cornea with respect to a standardized surface.

5. Use of the biometric recognition device, in accordance with claim 4, for the authentication of an individual.

6. Use of the biometric recognition device, in accordance with claim 4, for the identification of an individual.

## Patentansprüche

1. Verfahren für die biometrische Erkennung umfassend (a) Aufnahme eines Bildes der hinteren Fläche der Hornhaut und (b) Quantifizierung der Unregelmäßigkeiten einer Karte der genannten hinteren Fläche der Hornhaut in Bezug auf eine standardisierte Fläche, um charakteristische und differenzierende Parameter der hinteren Fläche der Hornhaut in jedem Individuum zu erhalten.

2. Verfahren für die biometrische Erkennung nach Anspruch 1, in Kombination mit einem anderen Verfahren für die biometrische Erkennung durch Verwendung von okularen Parametern, die aus einer photographischen Aufzeichnung entnommen worden sind, die erhalten wurde, um die Unregelmäßigkeiten der Flächenkarte des zweiten okularen dioptrischen Apparats zu quantifizieren.

3. Verfahren für die biometrische Erkennung nach Anspruch 2, wobei sich andere okulare Parameter sowohl auf die Hornhaut, wie Hornhautradii, dioptrische Stärke oder Brechkraft der Hornhaut, als auch auf die Netzhaut oder Iris beziehen.

4. Vorrichtung für die biometrische Erkennung umfassend eine rotierende Scheimpflug-Kamera und ein System für die Quantifizierung von Unregelmäßigkeiten der hinteren Fläche der Hornhaut in Bezug auf eine standardisierte Fläche.

5. Verwendung der Vorrichtung für die biometrische Erkennung nach Anspruch 4 für die Authentifizierung eines Individuums.

6. Verwendung der Vorrichtung für die biometrische Erkennung nach Anspruch 4 für die Identifizierung eines Individuums.

## Revendications

1. Procédé de reconnaissance biométrique qui comprend (a) la capture d'une image de la surface postérieure de la cornée et (b) la quantification des irrégularités d'une carte de ladite surface postérieure de la cornée par rapport à une surface normalisée pour l'obtention de paramètres caractéristiques et de différenciation de la surface postérieure de la cornée chez chaque individu.

2. Procédé de reconnaissance biométrique selon la revendication 1, en combinaison avec n'importe quel procédé de reconnaissance biométrique par le biais de l'utilisation de paramètres oculaires extraits d'un enregistrement photographique obtenu pour quantifier les irrégularités de la carte de surface du deuxième dioptre oculaire.

3. Procédé de reconnaissance biométrique selon la revendication 2, dans lequel d'autres paramètres oculaires sont aussi bien en rapport avec la cornée, tel que les rayons cornéens, la puissance dioptrique ou la puissance réfractive de la cornée, qu'avec la rétine ou l'iris.

4. Dispositif de reconnaissance biométrique comprenant une caméra rotative de Scheimpflug et un système de quantification des irrégularités de la surface postérieure de la cornée par rapport à une surface normalisée.

5. Utilisation du dispositif de reconnaissance biométrique selon la revendication 4, pour l'authentification d'un individu.

6. Utilisation du dispositif de reconnaissance biométrique selon la revendication 4, pour l'identification d'un individu.
